# EUROPÄISCHE PATENTSCHRIFT

(11) **EP 1 917 866 B1**
(45) Veröffentlichungstag und Bekanntmachung des Hinweises auf die Patenterteilung: **09.02.2011**
(21) Anmeldenummer: 07019703.3
(22) Anmeldetag: 09.10.2007
(51) Int. Cl.: A23L 1/00, A23L 1/29, A61P 1/00

(54) **Verfahren zur Herstellung von insbesondere tiefgekühlter, portionierter Brei- oder Pürreekost**
Method for manufacturing in particular frozen mashed or pureed foods in portions
Procédé de fabrication, en particulier de purée congelée et en portions

(30) Priorität: 13.10.2006 AT 17072006
(43) Veröffentlichungstag der Anmeldung: 07.05.2008
(73) Patentinhaber: Resama GmbH, 66424 Homburg (DE)
(72) Erfinder: Reier, Georg, 66424 Homburg Saar (DE)
(74) Vertreter: Hofinger, Stephan

(56) Entgegenhaltungen:
- EP-A- 0 966 887
- JP-A- 11 075 769
- JP-A- 55 118 373
- JP-A- 59 166 063
- US-A1- 2004 258 823
- US-B1- 6 676 986
- HOTALING D L: "NUTRITIONAL CONSIDERATIONS FOR THE PUREED DIET TEXTURE IN DYSPHAGIC ELDERLY" DYSPHAGIA, SPRINGER INTERNATIONAL INC., NEW YORK, NY, US, Bd. 7, Nr. 2, 1992, Seiten 81-85, XP009008648

## Beschreibung

Die Erfindung betrifft ein Verfahren zur Herstellung von insbesondere tiefgekühlter, portionierter Brei- oder Püreekost, die zum Verzehr im erwärmten Zustand bestimmt ist, bei dem zumindest ein erwärmter und mechanisch zerkleinerter, insbesondere pürierter und/oder passierter Trägerrohstoff, beispielsweise Gemüse, Fleisch, Fisch, Getreide oder Teigwaren, mit weiteren Ingredienzien vermischt wird und in weiterer Folge diese Mischung gekühlt und portioniert wird.

Die Herstellung tiefgekühlter, portionierter Püreekost ist an sich bekannt z.B. aus US 6,676,986; JP 55 118 373; JP 59 166 063; JP 11 075 769; EP-A 0 966 887. Dabei werden in der Regel die Rohstoffe wie Früchte, Gemüse, Fleisch und Fisch gegart und zerkleinert, oder umgekehrt, und im Anschluss daran feinst passiert, bevor die derart behandelten Rohstoffe in Formen gefüllt tiefgekühlt werden.

Tiefgekühlte und portionierte Püreekost bietet dem Verbraucher entscheidende Zeit- und Handlingvorteile, da Vorarbeiten wie Waschen, Putzen, Kochen, Zerkleinern und Nacharbeiten wie das zeitraubende Reinigen von Püriergeräten komplett entfällt. Die tiefgekühlten Portionen müssen nur mehr aufgetaut, individuell gewürzt und zubereitet werden.

Als nachteilig an den bekannten Püreekostformen hat sich jedoch herausgestellt, dass diese nach dem Auftauen beim Erwärmungsprozess ihre Form verlieren und sich verflüssigen, sodass der Verbraucher abhängig von den verwendeten Rohstoffen nur mehr unterschiedlich färbige Breie auf seinem Teller vorfindet, die eine Unterscheidung in Hauptspeise und Beilage nicht mehr zulassen.

Die Erfindung hat es sich daher zur Aufgabe gemacht, ein neuartiges Verfahren zur Herstellung von vorzugsweise tiefgekühlter, portionierter Püreekost anzugeben, mit dem die vorbeschriebenen Nachteile vermieden werden können, sodass die Püreekost auch nach dem Auftauen und Erwärmen ein optisch ansprechendes Aussehen hat.

Die Erfindung löst diese Aufgabe durch ein neuartiges Verfahren mit den Schritten a) bis e) gemäß dem Kennzeichen des Anspruches 1.

Durch die zweimalige Erwärmung der Flüssig-, Brei- oder Püreekomponenten können diese in formstabile Portionsgrößen geformt werden. In anderen Worten ausgedrückt wird beim erfindungsgemäßen Verfahren im Schritt a) die Struktur der Trägerrohstoffe gelöst und gemäß Schritt b) zerstört, bevor im Schritt c) Protein beigemengt wird, wodurch sich bei einer nochmaligen Erwärmung dieser Mischung gemäß Schritt d) aufgrund der Bindefähigkeit des Proteins eine neue formstabile Struktur ausbildet, die jedoch immer noch die Eigenschaften von Brei oder Püree aufweist, d.h. die formstabile Püreekost schmilzt sozusagen im Mund und muss nicht zerkaut werden, wodurch sich die mit dem neuartigen Verfahren hergestellte Püreekost insbesondere für Verbraucher mit Schluckbeschwerden eignet.

Für ein sicheres Auflösen dieser Primärstruktur und aus bakteriologischer Sicht hat es sich dabei gemäß einer Ausführungsform der Erfindung als vorteilhaft herausgestellt, wenn der Trägerrohstoff gemäß Schritt a) auf eine Kerntemperatur von etwa 75°C erwärmt wird.

Günstigerweise wird der Trägerrohstoff gegart, wobei die Erwärmung des Trägerrohstoffes im Wasserbad, mittels Dampf oder mit Heißluft erfolgen kann. Als besonders günstig für die nachfolgenden Verarbeitungsschritte hat es sich dabei herausgestellt, wenn die verschiedenen Trägerrohstoffe ohne Würzung im Dampf schonend bei Temperaturen von etwa 80 bis 90°C gegart werden, bis sie eine Kerntemperatur von 75°C erreichen, bevor der Trägerrohstoff gemäß Schritt b) auf eine Temperatur unter 25°C, vorzugsweise unter 15°C abgekühlt wird.

Das sofortige Abkühlen der Trägerrohstoffe soll zum einen sicherstellen, dass die Nährstoffdichte der einzelnen Trägerrohstoffe nicht verloren geht und zum anderen den Zerkleinerungsprozess der Trägerrohstoffe erleichtern, wobei es sich als vorteilhaft herausgestellt hat, wenn der Trägerrohstoff auf eine Temperatur von etwa 5°C abgekühlt, vorzugsweise schackgekühlt wird.

In Abhängigkeit vom gewünschten Endprodukt kann dem erwärmten und abgekühlten Trägerrohstoff vor und/oder beim Zerkleinern Flüssigkeit zugegeben werden, wobei gemäß einer weiteren Ausführungsform der Erfindung als Flüssigkeit Wasser, Fond, Sahne, Milch oder Öf, oder eine Mischung davon, zugegeben wird.

Gemäß einem Ausführungsbeispiel der Erfindung ist vorgesehen, dass das Verhältnis Trägerrohstoff zu Flüssigkeit zwischen 30 : 70 und 10 : 90, vorzugsweise etwa 20 : 80, beträgt In diesem Fall spricht man von Flüssigrohstoffen. Als Trägerrohstoffe eigenen sich dabei besonders Fisch, Schweinefleisch, Geflügel, Wild oder Gemüse.

Ein anderes Ausführungsbeispiel sieht vor, dass das Verhältnis Trägerrohstoff zu Flüssigkeit zwischen 70 : 30 und 40 : 60, vorzugsweise etwa 50 : 50, beträgt. In diesem Fall spricht man von Breirohstoffen, wobei sich als Trägerrohstoffe hauptsächlich Fleisch, Fisch und Teigwaren eignen.

Wird der Trägerrohstoff ohne Flüssigkeitszugabe zerkleinert, spricht man von Püreerohstoffen. Diese Püreekostform wird hauptsächlich bei Gemüse angewendet, da bei Gemüse der Wasseranteil beim Trägerrohstoff ausreichend hoch ist.

Um sicherzustellen, dass das Endprodukt keine störenden Klümpchen oder zusammenhängende Fasern beinhaltet, sieht ein weiteres Ausführungsbeispiel der Erfindung vor, dass die durchschnittliche Schnittgutgröße des abgekühlten und zerkleinerten Trägerrohstoffs unter 1,5 mm, vorzugsweise unter 1 mm liegt, wobei es sich als besonders günstig herausgestellt hat, wenn die durchschnittliche Schnittgutgröße unter 0,7 mm, vorzugsweise bei etwa 0,3 mm liegt.

Grundsätzlich kann die Zerkleinerung mit jedem dafür geeigneten Gerät durchgeführt werden. Eine bevorzugte Ausführungsform der Erfindung sieht jedoch vor, dass die Zerkleinerung des Trägerrohstoffes mittels eines Mikrokutters erfolgt. Ein geeigneter Mikrokutter ist beispielsweise der Stephan MCHD90V Mikrokutter.

Durch die Erwärmung während des Garens und die darauf folgende Zerkleinerung ist das in dem Trägerrohstoff enthaltende Protein denaturiert, das heißt, die Bindefähigkeit des Produktes bei einer Formgebung und Erwärmung ist nicht mehr gegeben. Da die Bindefähigkeit des Proteins bei der Produktion der Brei- oder Püreekostform benötigt wird, sieht ein weiteres Ausführungsbeispiel der Erfindung vor, dass der mit oder ohne Flüssigkeitszugabe zerkleinerte Trägerrohstoff mit Protein vermengt und zu einer homogenen brei- oder püreeförmigen Mischung vermischt wird. Weiters ist es möglich, dass der den zerkleinerten Trägerrohstoff und Protein enthaltenden Mischung weitere Ingredienzien beigemengt werden, wobei die Beimengung des Proteins und der weiteren Ingredienzien vorzugsweise in einem Arbeitsschritt erfolgt.

Erfindungsgemäß wird als Protein vorzugsweise pulverförmiges Hühnereiweiß verwendet, wobei eine besonders gute Bindefähigkeit und eine hohe biologische Wertigkeit erreicht wird, wenn als Protein eine Mischung von vorzugsweise pulverförmigem Hühnereiweiß und Lactalbumin verwendet wird.

Dabei sieht eine weitere Ausführungsform der Erfindung vor, dass das Verhältnis von Hühnereiweiß zu Lactalbumin zwischen 70 : 30 und 90 : 10, vorzugsweise bei etwa 80 : 20, liegt.

Neben der guten Bindefähigkeit und der hohen biologischen Wertigkeit wird durch die Verwendung von Hühnereiweiß weiters erreicht werden, dass die fertige Brei- oder Püreekost zwar formstabil ist, die Eigenschaften von Brei oder Püree aber beibehält, das heißt, dass aufgetaute und erwärmte Produkt kann vom Verbraucher mit der Zunge oder dem Gaumen zerdrückt werden und ist deshalb besonders für Verbraucher mit Schluckbeschwerden geeignet.

Die weiteren Ingredienzien können gemäß einer weiteren Ausführungsform der Erfindung Bindemittel, Geliermittel, Gewürze oder Sättigungsbeilagen oder eine Mischung davon umfassen.

Dabei hat es sich als vorteilhaft herausgestellt, wenn als Bindemittel modifizierte Stärke verwendet wird, da modifizierte Stärke gegenüber physikalischen oder enzymatischen Stärken mehrere Vorteile aufweist. So ist modifizierte Stärke kaltquellend, hitzestabil, säurestabil, scherstabil und gefrier- und auftaustabil. Zusätzlich verfügt modifizierte Stärke über ein besseres Fließverhalten bei der Abfüllung. Mit Hilfe der Stärke wird die Konsistenz der den zerkleinerten Trägerrohstoff und die weiteren Ingredienzien enthaltenden Mischung eingestellt, um eine bessere Abfülleigenschaft der Mischung zu erlagen. Zusätzlich verhindert der Einsatz von Bindemittel eine Entmischung der Rohstoffe, was aufgrund des niedrigen ph-Wertes, der zwischen 4 und 6,5 variieren kann, ohne Zugabe von Bindemittel und Geliermittel durchaus passieren kann.

Gemäß einem weiteren Ausführungsbeispiel der Erfindung ist vorgesehen, dass als Geliermittel Rindergelatine mit einem Bloomgrad zwischen 150 und 270, vorzugsweise zwischen 180 und 240 verwendet wird. Derartige Gelatine verhindert eine Synerese bei der Erhitzung und Abkühlung der einzelnen Endprodukte und fördert dazu die Ausbildung einer Kollagenstruktur, die eine Verformung des Endproduktes im warmen oder gekühlten Zustand verhindert, wodurch für den Endverbraucher ein perfekter Arbeitsablauf gewährleistet werden kann.

Zur Herstellung vollwertiger Mahlzeiten kann es sinnvoll sein, wenn gemäß einer weiteren Ausführungsform der Erfindung als Sättigungsbeilage Frischkäse, Grieß, Reis, Reisflocken oder Topfen oder eine Mischung davon verwendet wird.

Ebenso wie für die Zerkleinerung kann man auch für den Mischvorgang alle geeigneten Geräte verwendet werden. Versuche der Anmelderin haben jedoch ergeben, dass ein besonders günstiges Mischergebnis mit einem Taumelmischer erzielt werden kann, Dabei wird der Mischbehälter zu ungefähr 2/3 seines Volumens mit dem jeweiligen Mischgut gefüllt und während einer vorgewählten Zeit, beispielsweise zwischen 5 und 10, vorzugsweise zwischen 8 und 10 Minuten über Kopf gedreht. Die Schräglage des Mischbehälters verstärkt dabei zusammen mit dem an der Unterseite des Deckels montierten Mischkreuz den Mischeffekt. Auf diese Weise wird das Mischprodukt geschont und einwandfrei bearbeitet. Ein geeigneter Taumelischer ist beispielsweise der Fuchs-Mixomat-Taumeimischer.

Ist der Mischvorgang, bei dem das Mischgut eine Temperatur von ca. 10 bis 15 ° Celsius aufweist, abgeschlossen, bieten sich im Wesentlichen zwei Möglichkeiten der weiteren Verarbeitung an.

So sieht ein Ausführungsbeispiel der Erfindung vor, dass die brei- oder püreeförmige Mischung vor dem Erwärmungsprozess gemäß Schritt d) in einen temperaturbeständigen Plastikdarm gefüllt wird und die Erwärmung der in den Plastikdarm abgefüllten brei- oder püreeförmigen Mischung und die Überführung derselben in eine formstabile Brei- oder Püreekost im Wasserbad oder mittels Dampf erfolgt. Das Befüllen des hitzebeständigen Plastikdarms, dessen Durchmesser zwischen 3 und 15 cm variieren kann, kann beispielsweise mittels eines Vakuumfüllers erfolgen.

Gemäß eines bevorzugten Ausführungsbeispiels der Erfindung ist vorgesehen, dass der die erwärmte und formstabile Brei- oder Püreekost enthaltende Plastikdarm zwischen 5 und 20 min, vorzugsweise zwischen 10 und 15 min, bei einer zwischen 5 und 15°C, vorzugsweise bei 10°C liegenden Temperatur gekühlt und im Anschluss daran portioniert wird und die Portionen tiefgekühlt, vorzugsweise schockgekühlt werden.

Ein anderes Ausführungsbeispiel der Erfindung sieht vor, dass die brei- oder püreeförmige Mischung vor dem Erwärmungsprozess gemäß Schritt d) in temperaturbeständige Formen, vorzugsweise Silikonformen, gefüllt wird und im Anschluss daran die Erwärmung der in die Formen gefüllten brei- oder püreeförmigen Mischung und die Überführung derselben in eine formstabile Brei- oder Püreekost mittels Heißluft, in die Feuchtigkeit eingesprüht wird, erfolgt bevor die die nach dem Erwärmungsprozess formstabile Brei- oder Püreekost enthaltenden Formen tiefgekühlt, vorzugsweise schockgekühlt, werden.

Unabhängig davon, ob die gegarten Trägerrohstoffe mit oder ohne Flüssigkeitszugabe zerkleinert werden, ob außer Protein weitere Ingredienzien beigemengt werden und ob die Abfüllung der Rohmasse in einen Kunstdarm oder in Silikonformen erfolgt, besteht eine wesentliche Grundidee der Erfindung jedenfalls darin, dass die Erwärmungszeit t₂ gemäß Schritt d) kürzer als die Erwärmungszeit t₁ gemäß Schritt a) ist. Eine schonende zweite Erwärmung kann dabei sichergestellt werden, wenn die Erwärmungszeit t₂ mehr als 25 % der Erwärmungszeit t₁ beträgt, wobei sich als günstig herausgestellt hat, wenn die Erwärmungszeit t₂ zwischen 50% und 80 %, vorzugsweise zwischen 60% und 70%, der Erwärmungszeit t₁ beträgt.

Nachfolgend wird ein Ausführungsbeispiel einer tiefgekühlten portionierten Brei- und Püreekost, die insbesondere nach dem erfindungsgemäßen Verfahren hergestellt wurde und sich dadurch auszeichnet, dass sie nach dem Auftauen und Erwärmen eine formstabile Proteinstruktur aufweist, beschrieben:
20 kg Rinderbrei (Rindfleisch und Rindsfond))
13 kg Sahne mit einem Fettgehalt von 10 bis 36 %, idealerweise von 20 bis 25 %
7 kg Frischkäse mit einer Trockenmasse von 45 %
0,4 kg Gulaschgewürz
0,03 modifizierte Stärke (kaltquellend) auf Basis von Mais, Reis, Kartoffel oder Getreide
0.1 kg Proteine in Form von 80 % Hühnereiweißpulver und 20 % Lactalbumin
1 kg Rindergelatine (240 Blom)

Bei diesem Ausführungsbeispiel handelt es sich um Rindergulasch, bei dem der Anteil des Trägerrohstoffes am Endprodukt ca. 80 % beträgt. Dieser Prozentsatz kann je nach Ausgangsrohstoff und gewünschtem Endprodukt zwischen 50 und 90 % variieren.

Zur Herstellung des Endproduktes wird das Rindfleisch mit 80 bis 90 °C heißem Dampf oder 120 °C heißer Heißluft auf 75 °C Kerntemperatur erhitzt und danach sofort auf 5 °C abgekühlt, sodass die Nährstoffdichte nicht verloren geht. Anschließend wird das gekochte Rinderfleisch zusammen mit Rindsfond in einem Stephan-Mikrokutter zerkleinert. Diese zerkleinerte breiförmige Mischung wird dann zusammen mit dem Frischkäse, dem Gulaschgewürz, der modifizierten Stärke, dem Protein und der Rindergelatine in einem Fuchs-Mixomat-Taumelmischer vermischt.

Nach dem Mischvorgang wird die produzierte Mischung mit einem Vakuumfüller in einen hitzebeständigen Plastikdarm abgefüllt und im Wasserbad auf eine Kerntemperatur von 75 °C erwärmt. Diese durch die Erwärmung formstabilen Darmstangen, deren Durchmesser zwischen 5 und 14 cm liegen kann, werden in weiterer Folge kurz überkühlt, in Scheiben von 1,5 bis 4 cm geschnitten und danach tiefgekühlt oder sofort tiefgekühlt und danach in Scheiben geschnitten.

Eine ganz allgemeine Rezeptur zur Herstellung formstabiler Brei- oder Püreekost umfasst die Schritte
1. Herstellung der Flüssig-, Brei- oder Püreekostform (Fleisch dämpfen, mit Flüssigkeit versetzen und im Mikrocutter zerkleinern)
2. Sahne, Frischkäse, Topfen, Joghurt oder Mascarpone zugeben
3. Gelatine in der 15-fachen Wassermenge einweichen und erhitzen
4. die Trockenprodukte, beispielsweise Eiweiß, Stärke und/oder Gewürze gut mischen und
5. die ganzen Zutaten mit dem Mixomat mischen
6. den Darm im lauwarmen Wasser 15 Minuten einweichen und die Masse mit dem Vakuumfüller einfüllen
7. im Wasserbad, Dampf- oder Heißluftofen bei 80 bis 90°C erhitzen (Formgebung)
8. kurz, 10 bis 15 Minuten, bei ca. 10 °G kühlen
9. in Portionen schneiden und im Spiralfroster schockkühlen oder
9a zuerst schockkühlen und danach in Portionen schneiden.
10. in beispielsweise 1 bis 5 kg große Gebinde verpacken.

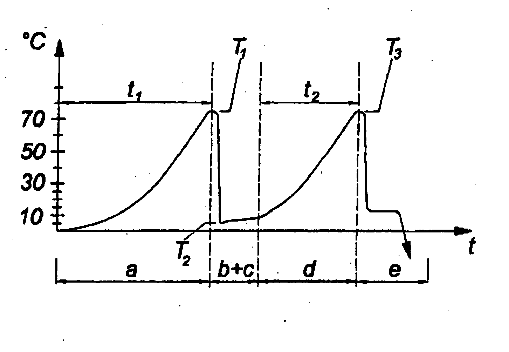

Weitere Vorteile und Einzelheiten werden unter Bezugnahme auf die Zeichnung näher erläutert.

Aus der einzigen Figur ist der neuartige Temperaturverlauf des erfindungsgemäßen Herstellungsverfahrens deutlich ersichtlich. Während des Erwärmens gemäß Schritt a) des erfindungsgemäßen Verfahrens steigt die Kerntemperatur T₁ des Trägerrohstoffes auf 75 °C an und sinkt durch die nachfolgende Abkühlung rasch auf eine Temperatur T₂ von 5 °C ab. In weiterer Folge und zwar während der Schritte b) und c) pendelt sich die Temperatur auf einen Wert um ca. 10 °C ein, bevor sie bei der Erwärmung gemäß Schritt d) des erfindungsgemäßen Verfahrens wieder auf eine Kerntemperatur T₃ von 75 °C ansteigt um beim Abkühlen und Einfrieren gemäß Schritt e) in den Minusbereich abzusinken.

Die mit dem erfindungsgemäßen Verfahren produzierte tiefgekühlte, portionierte Brei- oder Püreeformkost eignet sich, wie bereits erwähnt, insbesondere für Verbraucher mit Schluckbeschwerden und kann in der Mikrowelle durch Induktion mittels Dampf- oder Heißluftöfen auf eine Kerntemperatur von 75 bis 80 °C gebracht und regeneriert werden. Bei dieser Regenerierung bleiben die Produkte formstabil und erhalten ihre Brei- oder Püreekoststruktur. Bei von der Anmelderin durchgeführten Sensoriktestreihen wurden die Strukturvorgaben für Verbraucher mit Schluckbeschwerden von nach dem neuartigen Verfahren hergestellten Produkten immer erreicht. Außerdem wurden für Heimservice-Anbieter spezielle Testreihen mit Induktionsplatten durchgeführt, wobei Temperaturen von über 100 °C erreicht wurden und die Produkte überraschenderweise trotzdem formstabil blieben. Weitere Versuche der Anmelderin haben gezeigt, dass die derart hergestellte Brei-oder Püreekostform auch beim Regenerieren mit niederen Temperaturen zwischen 35 und 60 °C stabil bleiben und nicht zerfallen.

Es versteht sich von selbst, dass die Erfindung nicht auf das beschriebene Ausführungsbeispiels beschränkt ist. Eine Grundidee der Erfindung besteht jedenfalls darin, die Trägerrohstoffe in einem ersten Schritt zu Garen, danach zu Zerkleinern und mit Protein zu vermengen und über einen zweiten Erwärmungsprozess wiederum eine Proteinstruktur aufzubauen.

## Patentansprüche

1. Verfahren zur Herstellung von insbesondere tiefgekühlter, portionierter Brei- oder Püreekost, die zum Verzehr im erwärmten Zustand bestimmt ist, bei dem zumindest ein erwärmter und mechanisch zerkleinerter, insbesondere pürierter und/oder passierter Trägerrohstoff, beispielsweise Gemüse, Fleisch, Fisch, Getreide oder Teigwaren, mit weiteren Ingredienzien vermischt wird und in weiterer Folge diese Mischung gekühlt und portioniert wird, **gekennzeichnet durch** folgende Schritte:
a) Erwärmen des Trägerrohstoffes auf eine Kerntemperatur T₁ von wenigstens 60°C, vorzugsweise wenigstens 70°C;
b) Abkühlen, vorzugsweise Schockkühlen des Trägerrohstoffes auf eine wesentlich niedrigere Temperatur T₂ und mechanisches Zerkleinern des Trägerrohstoffes;
c) Vermengen des zerkleinerten Trägerrohstoffes mit Protein und vermischen zu einer homogenen brei- oder püreeförmigen Mischung, wobei als Protein - vorzugsweise pulverförmiges - Hühnereiweiß oder eine Mischung von - vorzugsweise pulverförmigem - Hühnereiweiß und Lactalbulmin verwendet wird;
d) Überführen der brei- oder püreeförmigen Mischung in eine formstabile Brei- oder Püreekost **durch** Erwärmen auf eine Kerntemperatur T₃ von wenigstens 60°C, vorzugsweise wenigstens 70°C;
e) Abkühlen, vorzugsweise Schockkühlen der formstabilen Brei- oder Püreekost;

2. Verfahren nach Anspruch 1, **dadurch gekennzeichnet, dass** der Trägerrohstoff gemäß Schritt a) auf eine Kerntemperatur T₁ von etwa 75°C erwärmt wird.

3. Verfahren nach Anspruch 1 oder Anspruch 2, **dadurch gekennzeichnet, dass** der Trägerrohstoff gegart wird.

4. Verfahren nach einem der Ansprüche 1 bis 3, **dadurch gekennzeichnet, dass** die Erwärmung des Trägerrohstoffes im Wasserbad, mittels Dampf oder mit Heißluft erfolgt.

5. Verfahren nach einem der Ansprüche 1 bis 4, **dadurch gekennzeichnet, dass** der Trägerrohstoff gemäß Schritt b) auf eine Temperatur T₂ unter 25°C, vorzugsweise unter 15°C abgekühlt wird.

6. Verfahren nach Anspruch 5, **dadurch gekennzeichnet, dass** der Trägerrohstoff auf eine Temperatur T₂ von etwa 5°C abgekühlt wird.

7. Verfahren nach einem der Ansprüche 1 bis 6, **dadurch gekennzeichnet, dass** dem erwärmten und abgekühlten Trägerrohstoff vor und/oder beim Zerkleinern Flüssigkeit zugegeben wird.

8. Verfahren nach Anspruch 7, **dadurch gekennzeichnet, dass** als Flüssigkeit Wasser, Fond, Sahne, Milch oder Öl oder eine Mischung davon zugegeben wird.

9. Verfahren nach Anspruch 7 oder Anspruch 8, **dadurch gekennzeichnet, dass** das Verhältnis Trägerrohstoff zu Flüssigkeit zwischen 30 : 70 und 10 : 90, vorzugsweise etwa 20 : 80, beträgt.

10. Verfahren nach Anspruch 7 oder Anspruch 8, **dadurch gekennzeichnet, dass** das Verhältnis Trägerrohstoff : Flüssigkeit zwischen 70 : 30 und 40 : 60, vorzugsweise etwa 50 : 50, beträgt.

11. Verfahren nach einem der Ansprüche 1 bis 10, **dadurch gekennzeichnet, dass** die durchschnittliche Schnittgutgröße des abgekühlten und zerkleinerten Trägerrohstoffs unter 1,5 mm, vorzugsweise unter 1 mm liegt.

12. Verfahren nach Anspruch 11, **dadurch gekennzeichnet, dass** die durchschnittliche Schnittgutgröße unter 0,7 mm, vorzugsweise bei etwa 0,3 mm liegt.

13. Verfahren nach einem der Ansprüche 1 bis 12, **dadurch gekennzeichnet, dass** die Zerkleinerung des Trägerrohstoffes mittels eines Mikrokutters erfolgt.

14. Verfahren nach einem der Ansprüche 1 bis 13, **dadurch gekennzeichnet, dass** der den zerkleinerten Trägerstoff und Protein enthaltenden Mischung weitere Ingredienzien beigemengt werden, wobei die Beimengung des Proteins und der weiteren Ingredienzien vorzugsweise in einem Arbeitsschritt erfolgt.

15. Verfahren nach Anspruch 14, **dadurch gekennzeichnet, dass** die weiteren Ingredienzien Bindemittel, Geliermittel, Gewürze oder Sättigungsbeilagen oder eine Mischung davon umfassen.

16. Verfahren nach Anspruch 15, **dadurch gekennzeichnet, dass** als Bindemittel modifizierte Stärke verwendet wird.

17. Verfahren nach einem der Ansprüche 15 oder 16, **dadurch gekennzeichnet, dass** als Geliermittel Rindergelatine mit einem Bloomgrad zwischen 150 und 270, vorzugsweise zwischen 180 und 240 verwendet wird.

18. Verfahren nach einem der Ansprüche 15 bis 17, **dadurch gekennzeichnet, dass** als Sättigungsbeilage Frischkäse, Grieß, Reis, Reisflocken oder Topfen oder eine Mischung davon verwendet wird.

19. Verfahren nach einem der Ansprüche 1 bis 18, **dadurch gekennzeichnet, dass** - bei einer Verwendung einer Mischung von vorzugsweise pulverförmigem Hühnereiweiß und Lactalbulmin als Protein - das Verhältnis von Hühnereiweiß zu Lactalbumin zwischen 70 : 30 und 90 : 10, vorzugsweise bei etwa 80 : 20, liegt.

20. Verfahren nach einem der Ansprüche 1 bis 20, **dadurch gekennzeichnet, dass** die brei- oder püreeförmige Mischung vor dem Erwärmungsprozess gemäß Schritt d) in einen temperaturbeständigen Plastikdarm gefüllt wird.

21. Verfahren nach Anspruch 20, **dadurch gekennzeichnet, dass** die Erwärmung der in den Plastikdarm abgefüllten brei- oder püreeförmigen Mischung und die Überführung derselben in eine formstabile Brei- oder Püreekost im Wasserbad oder mittels Dampf erfolgt.

22. Verfahren nach Anspruch 20 oder Anspruch 21, **dadurch gekennzeichnet, dass** der die erwärmte und formstabile Brei- oder Püreekost enthaltende Plastikdarm zwischen 5 und 20 min, vorzugsweise zwischen 10 und 15 min, bei einer zwischen 5 und 15°C, vorzugsweise bei 10°C liegenden Temperatur gekühlt und im Anschluss daran portioniert wird und die Portionen tiefgekühlt, vorzugsweise schockgekühlt werden.

23. Verfahren nach einem der Ansprüche 1 bis 19, **dadurch gekennzeichnet, dass** die brei- oder püreeförmige Mischung vor dem Erwärmungsprozess gemäß Schritt d) in temperaturbeständige Formen, vorzugsweise Silikonformen, gefüllt wird.

24. Verfahren nach Anspruch 23, **dadurch gekennzeichnet, dass** die Erwärmung der in die Formen gefüllten brei- oder püreeförmigen Mischung und die Überführung derselben in eine formstabile Brei- oder Püreekost mittels Heißluft, in die Feuchtigkeit eingesprüht wird, erfolgt.

25. Verfahren nach Anspruch 23 oder 24, **dadurch gekennzeichnet, dass** die die nach dem Erwärmungsprozess formstabile Brei- oder Püreekost enthaltenden Formen tiefgekühlt, vorzugsweise schockgekühlt, werden.

26. Verfahren nach einem der Ansprüche 1 bis 25, **dadurch gekennzeichnet, dass** die Erwärmungszeit t₂ gemäß Schritt d) kürzer als die Erwärmungszeit t₁ gemäß Schritt a) ist.

27. Verfahren nach Anspruch 26, **dadurch gekennzeichnet, dass** die Erwärmungszeit t₂ mehr als 25 % der Enrvärmungszeit t₁ beträgt.

28. Verfahren nach Anspruch 26 oder 27, **dadurch gekennzeichnet, dass** die Erwärmungszeit t₂ zwischen 50% und 80 %, vorzugsweise zwischen 60% und 70%, der Erwärmungszeit t₁ beträgt.

## Claims

1. A process for the production of in particular deep-frozen, portioned mashed or pureed food which is intended for consumption in the heated condition, in which at least one heated and mechanically minced, in particular pureed and/or strained carrier raw material, for example vegetables, meat, fish, cereal or pasta is mixed with further ingredients and subsequently said mixture is cooled and portioned, **characterised by** the following steps:
a) heating the carrier raw material to a core temperature T, of at least 60°C, preferably at least 70°C;
b) cooling, preferably shock cooling, of the carrier raw material to a substantially lower temperature T₂ and mechanically mincing the carrier raw material;
c) mixing the minced carrier raw material with protein and mixing to give a homogeneous mashed or pureed mixture, wherein chicken albumin - preferably in powder form - or a mixture of chicken albumin - preferably in powder form - and lactalbumin is used as the protein;
d) converting the mashed or pureed mixture into a mashed or pureed food which is stable in respect of shape by heating to a core temperature T₃ of at least 60°C, preferably at least 70°C; and
e) cooling, preferably shock cooling, of the mashed or pureed food which is stable in respect of shape.

2. A process according to claim 1 **characterised in that** the carrier raw material according to step a) is heated to a core temperature T₁ of about 75°C.

3. A process according to claim 1 or claim 2 **characterised in that** the carrier raw material is cooked.

4. A process according to one of claims 1 to 3 **characterised in that** the step of heating the carrier raw material is effected in a water bath, by means of steam or with hot air.

5. A process according to one of claims 1 to 4 **characterised in that** the carrier raw material in accordance with step b) is cooled to a temperature T₂ of below 25°C, preferably below 15°C.

6. A process according to claim 5 **characterised in that** the carrier raw material is cooled to a temperature T₂ of about 5°C.

7. A process according to one of claims 1 to 6 **characterised in that** liquid is added to the heated and cooled carrier raw material before and/or upon mincing.

8. A process according to claim 7 **characterised in that** water, meat juices, cream, milk or oil or a mixture thereof is added as the liquid.

9. A process according to claim 7 or claim 8 **characterised in that** the ratio of carrier raw material to liquid is between 30:70 and 10:90, preferably about 20:80.

10. A process according to claim 7 or claim 8 **characterised in that** the ratio of carrier raw material to liquid is between 70:30 and 40:60, preferably about 50:50.

11. A process according to one of claims 1 to 10 **characterised in that** the average cut material size of the cooled and minced carrier raw material is below 1.5 mm, preferably below 1 mm.

12. A process according to claim 11 **characterised in that** the average cut material size is below 0.7 mm, preferably about 0.3 mm.

13. A process according to one of claims 1 to 12 **characterised in that** mincing of the carrier raw material is effected by means of a microcutter.

14. A process according to one of claims 1 to 13 **characterised in that** further ingredients are added to the mixture containing the minced carrier material and protein, wherein the addition of the protein and the further ingredients is preferably effected in one working step.

15. A process according to claim 14 **characterised in that** the further ingredients include binding agent, gelling agent, spices or saturation supplements or a mixture thereof.

16. A process according to claim 15 **characterised in that** modified starch is used as the binding agent.

17. A process according to one of claims 15 and 16 **characterised in that** cattle gelatine with a Bloom degree of between 150 and 270, preferably between 180 and 240, is used as the gelling agent.

18. A process according to one of claims 15 to 17 **characterised in that** cream cheese, semolina, rice, rice flakes or quark or a mixture thereof is used as the saturation supplement.

19. A process according to one of claims 1 to 18 **characterised in that** - when using a mixture of preferably powder-form chicken albumin and lactalbumin as protein - the ratio of chicken albumin to lactalbumin is between 70:30 and 90:10, preferably at about 80:20.

20. A process according to one of claims 1 to 19 **characterised in that** the mashed or pureed mixture is filled into a temperature-resistant plastic case prior to the heating process in accordance with step d).

21. A process according to claim 20 **characterised in that** heating of the mashed or pureed mixture introduced into the plastic case and conversion thereof into a mashed or pureed food which is stable in respect of shape is effected in a water bath or by means of steam.

22. A process according to claim 20 or claim 21 **characterised in that** the plastic case containing the heated mashed or pureed food which is stable in respect of shape is cooled for between 5 and 20 minutes, preferably between 10 and 15 minutes, at a temperature between 5 and 15°C, preferably at 10°C, and is then portioned and the portions are deep-frozen, preferably shock-cooled.

23. A process according to one of claims 1 to 19 **characterised in that** the mashed or pureed mixture is filled into temperature-resistant moulds, preferably silicone moulds, prior to the heating process in accordance with step d).

24. A process according to claim 23 **characterised in that** heating of the mashed or pureed mixture filled into the moulds and conversion thereof into a mashed or pureed food which is stable in respect of shape is effected by means of hot air into which moisture is sprayed.

25. A process according to claim 23 or claim 24 **characterised in that** the moulds containing the mashed or pureed food which is stable in respect of shape after the heating process are deep-frozen, preferably shock-cooled.

26. A process according to one of claims 1 to 25 **characterised in that** the heating time t₂ in accordance with step d) is shorter than the heating time t, in accordance with step a).

27. A process according to claim 26 **characterised in that** the heating time t₂ is more than 25% of the heating time t₁.

28. A process according to claim 26 or claim 27 **characterised in that** the heating time t₂ is between 50% and 80%, preferably between 60% and 70%, of the heating time t₁.

## Revendications

1. Procédé de fabrication d'une bouillie ou d'une purée alimentaire par portions, en particulier congelée, destinée à la consommation à l'état chauffé, dans lequel on mélange au moins une matière première formant support, chauffée et mécaniquement broyée, en particulier transformée en purée et/ou passée, par exemple des légumes, de la viande, du poisson, des céréales ou des pâtes, avec d'autres ingrédients, puis on refroidit et subdivise ce mélange en portions, **caractérisé par** les étapes suivantes :
a) chauffage de la matière première formant support à une température T₁ d'au moins 60°C, de préférence d'au moins 70°C ;
b) refroidissement, de préférence par refroidissement rapide, de la matière première formant support à une température T₂ nettement plus basse, et broyage mécanique de la matière première formant support ;
c) mélange de la matière première formant support ainsi broyée avec des protéines, et mélange pour former un mélange homogène, à structure de bouillie ou de purée, ce pour quoi on utilise en tant que protéines du blanc d'oeuf de poule - de préférence en poudre - ou un mélange de blanc d'oeuf de poule - de préférence en poudre - et de lactalbumine ;
d) conversion du mélange à structure de bouillie ou de purée en une bouillie ou une purée alimentaire de forme stable, par chauffage à une température de coeur T₃ d'au moins 60°C, de préférence d'au moins 70°C ;
e) refroidissement, de préférence refroidissement rapide, de la bouillie ou de la purée alimentaire de forme stable.

2. Procédé selon la revendication 1, dans lequel on chauffe la matière première formant support selon l'étape a) à une température de coeur T₁ d'environ 75°C.

3. Procédé selon la revendication 1 ou la revendication 2, **caractérisé en ce que** la matière première formant support est soumise à une cuisson.

4. Procédé selon l'une des revendications 1 à 3, **caractérisé en ce que** le chauffage de la matière première formant support a lieu au bain-marie, à l'aide de vapeur ou d'air chaud.

5. Procédé selon l'une des revendications 1 à 4, **caractérisé en ce que** la matière première formant support selon l'étape b) est refroidie à une température T₂ inférieure à 25°C, de préférence inférieure à 15°C.

6. Procédé selon la revendication 5, **caractérisé en ce que** la matière première formant support est refroidie à une température T₂ d'environ 5°C.

7. Procédé selon l'une des revendications 1 à 6, **caractérisé en ce que** la matière première formant support, chauffée et refroidie, est additionnée d'un liquide avant et/ou pendant le broyage.

8. Procédé selon la revendication 7, **caractérisé en ce qu'**on ajoute en tant que liquide de l'eau, un fond, de la crème, du lait ou de l'huile, ou un mélange de ceux-ci

9. Procédé selon la revendication 7 ou la revendication 8, **caractérisé en ce que** le rapport de la matière première formant support au liquide est compris entre 30:70 et 10:90, et est de préférence d'environ 20:80.

10. Procédé selon la revendication 7 ou la revendication 8, **caractérisé en ce que** le rapport matière première formant support:liquide est compris entre 70:30 et 40:60 et est de préférence d'environ 50:50.

11. Procédé selon l'une des revendications 1 à 10, **caractérisé en ce que** la granulométrie moyenne des morceaux découpés de la matière première formant support, refroidie et broyée, est inférieure à 1,5 mm, de préférence inférieure à 1 mm.

12. Procédé selon la revendication 11, **caractérisé en ce que** la granulométrie moyenne des morceaux découpés est inférieure à 0,7 mm, de préférence d'environ 0,3 mm.

13. Procédé selon l'une des revendications 1 à 12, **caractérisé en ce que** la fragmentation de la matière première formant support est effectuée à l'aide d'un microcutter.

14. Procédé selon l'une des revendications 1 à 13, **caractérisé en ce qu'**on ajoute au mélange contenant la matière formant support broyée et des protéines, d'autres ingrédients, l'addition des protéines et des autres ingrédients s'effectuant de préférence en une seule étape.

15. Procédé selon la revendication 14, **caractérisé en ce que** les autres ingrédients comprennent des liants, des gélifiants, des condiments ou des garnitures de satiété, ou un mélange de ceux-ci.

16. Procédé selon la revendication 15, **caractérisé en ce qu'**on utilise en tant que liant de l'amidon modifié.

17. Procédé selon l'une des revendications 15 ou 16, **caractérisé en ce qu'**on utilise en tant que gélifiant de la gélatine de bovin ayant un degré Bloom compris entre 150 et 270, de préférence entre 180 et 240.

18. Procédé selon l'une des revendications 15 à 17, **caractérisé en ce qu'**on utilise en tant que garniture de satiété du fromage frais, de la semoule, du riz, des flocons de riz ou du fromage blanc, ou un mélange de ceux-ci.

19. Procédé selon l'une des revendications 1 à 18, **caractérisé en ce que** - lors de l'utilisation, en tant que protéines, d'un mélange de blanc d'oeuf de poule de préférence en poudre et de lactalbumine - le rapport du blanc d'oeuf de poule à la lactalbumine est compris entre 70:30 et 90:10, et est de préférence d'environ 80:20.

20. Procédé selon l'une des revendications 1 à 19, **caractérisé en ce que** le mélange à structure de bouillie ou de purée est, avant l'opération de chauffage selon l'étape d), emballé dans un boyau en plastique résistant aux hautes températures.

21. Procédé selon la revendication 20, **caractérisé en ce que** le chauffage du mélange à structure de bouillie ou de purée, emballé dans le boyau en plastique, et sa conversion en une bouillie ou une purée alimentaire de forme stable, ont lieu au bain-marie, ou à l'aide de vapeur.

22. Procédé selon la revendication 20 ou la revendication 21, **caractérisé en ce que** le boyau en plastique contenant la bouillie ou la purée alimentaire, chauffée et de forme stable, est refroidi pendant 5 à 20 min, de préférence pendant 10 à 15 min, à une température comprise entre 5 et 15°C, de préférence de 10°C, puis est subdivisé, et les portions sont congelées, de préférence par refroidissement rapide.

23. Procédé selon l'une des revendications 1 à 19, **caractérisé en ce que** le mélange à structure de bouillie ou de purée est, avant l'opération de chauffage selon l'étape d), placé dans des moules résistant aux hautes températures, de préférence des moules en silicone.

24. Procédé selon la revendication 23, **caractérisé en ce que** le chauffage du mélange à structure de bouillie ou de purée, placé dans les moules, et leur conversion en une bouillie ou une purée alimentaire de forme stable, a lieu à l'aide d'air chaud, dans lequel on a pulvérisé de l'humidité.

25. Procédé selon la revendication 23 ou 24, **caractérisé en ce qu'**on congèle, de préférence par refroidissement rapide, les moules contenant la bouillie ou la purée alimentaire, de forme stable après l'opération de chauffage.

26. Procédé selon l'une des revendications 1 à 25, **caractérisé en ce que** le temps de chauffage t₂ selon l'étape d) est plus court que le temps de chauffage t₁ selon l'étape a).

27. Procédé selon la revendication 26, **caractérisé en ce que** le temps de chauffage t₂ est supérieur à 25 % du temps de chauffage t₁.

28. Procédé selon la revendication 26 ou 27, **caractérisé en ce que** le temps de chauffage t₂ est compris entre 50 et 80 %, de préférence entre 60 et 70 % du temps de chauffage t₁.
